# EUROPÄISCHE PATENTANMELDUNG

(11) **EP 2 221 092 A1**
(43) Veröffentlichungstag der Anmeldung: **25.08.2010**
(21) Anmeldenummer: 10001606.2
(22) Anmeldetag: 17.02.2010
(51) Int. Cl.: A63B 21/065, A63B 23/04, A61F 5/01, A61H 1/02, A63B 21/00, A63B 23/00

(54) **Orthopädische Vorrichtung**

(30) Priorität: 19.02.2009 DE 102009009681
(71) Anmelder: Wolf, Klaus, 79356 Eichstetten (DE)
(72) Erfinder: Wolf, Klaus, 79356 Eichstetten (DE)
(74) Vertreter: Maucher, Wolfgang

(57) **Zusammenfassung**

Die Erfindung betrifft eine orthopädische Vorrichtung zur Therapieunterstützung bei Gelenkbeschwerden des Hüftgelenks (16), welche Vorrichtung an demjenigen dem Hüftgelenk (16) zugeordneten Oberschenkel (17) anordenbar ist, an dessen einem Ende sich einer der Gelenkpartner des betroffenen Hüftgelenks (16) befindet, mit einer Stützeinrichtung (2), die durch wenigstens eine mit dem Oberschenkel lösbar (17) anordenbare Manschette (7) gebildet ist. Um eine einfach handhabbare orthopädische Vorrichtung (1) zur Verfügung zu haben, die bei einer durch Gelenkbeschwerden beeinträchtigten Person eine weitgehend schmerzfreie Verbesserung von Bewegungsabläufen, insbesondere von gelenkfernen Gliedmaßen, ermöglicht, wird vorgeschlagen, die Stützeinrichtung (2) mit einem Halteteil (3) zu verbinden, welches ein Gewichtselement (4) aufweist, dessen in einer Gebrauchsstellung auf den Oberschenkel (17) ausgeübtes Moment die Gelenkpartner des Hüftgelenks (16) trennt, und das Halteteil (3) mit einem ersten, von dem Oberschenkel (17) abstehenden Arm (5) zu versehen, entlang dessen das Gewichtselement (4) verlagerbar angeordnet ist.

## Beschreibung

Die Erfindung betrifft eine orthopädische Vorrichtung zur Therapieunterstützung bei Gelenkbeschwerden des Hüftgelenks, welche Vorrichtung an demjenigen dem Hüftgelenk zugeordneten Oberschenkel anordenbar ist, an dessen einem Ende sich einer der Gelenkpartner des betroffenen Hüftgelenks befindet, mit einer Stützeinrichtung, die durch wenigstens eine mit dem Oberschenkel lösbar anordenbare Manschette gebildet ist.

Derartige Vorrichtungen sind beispielsweise bereits aus der US 3 502 071 A bekannt und dienen beispielsweise dazu, bei posttraumatischen oder durch Verschleiß bedingten Beschwerden des Bewegungsapparates Oberschenkel ruhig zu stellen oder zu fixieren. Daher sind derartige Vorrichtungen nur bedingt geeignet, im Rahmen einer Therapie die Beweglichkeit von Personen mit etwa durch eine Arthrose verursachten Gelenkbeschwerden oder dergleichen Einschränkungen zu verbessern. Häufig sind solche Vorrichtungen auch nicht einfach handhabbar oder ihr Einsatz für den Patienten mit Schmerzen verbunden.

Es ist daher die Aufgabe der vorliegenden Erfindung, eine einfach handhabbare orthopädische Vorrichtung zur Verfügung zu stellen, die bei einer durch Gelenkbeschwerden beeinträchtigten Person eine weitgehend schmerzfreie Verbesserung von Bewegungsabläufen, insbesondere von gelenkfernen Gliedmaßen, ermöglicht.

Diese Aufgabe wird gelöst durch eine Vorrichtung der eingangs genannten Art, bei welcher die Stützeinrichtung mit einem Halteteil verbunden ist, welches ein Gewichtselement aufweist, dessen in einer Gebrauchsstellung auf den Oberschenkel ausgeübtes Moment die Gelenkpartner des Hüftgelenks trennt, und dass das Halteteil mit zumindest einem ersten, von dem Oberschenkel abstehenden Arm versehen ist, entlang dessen das Gewichtselement verlagerbar angeordnet ist.

Bei arthritischen Gelenkbeschwerden kommt es zu Knorpelschädigungen und nachfolgend auch zu Knochenveränderungen, bei welchen die durch Bewegungen oder Stellungen des Gelenks verursachten mechanischen Belastungen als schmerzhaft empfunden werden. Dies ist auch beim Hüftgelenk der Fall, bei welchem das Gelenk durch den Kopf des Oberschenkelknochens (Caput ossis femoris) und die Gelenkpfanne des Beckens (Acetabulum) gebildet wird und durch deren Zusammenspiel eine Belastungszone ausgebildet wird, in welcher der Gelenkknorpel unter einer Druckbelastung steht.

Durch das mittels der Vorrichtung auf die Körperextremität, also den Oberschenkelknochen, ausgeübte Moment können Gelenkkopf und Gelenkpfanne für den Patienten weitestgehend schmerzfrei getrennt und mithin zum einen die Schmerzursache zumindest zeitweilig vermieden, zum anderen die durch den Schmerz verursachte Bewegungshemmung wenigsten vermindert, wenn nicht beseitigt werden. In einer Gebrauchsstellung in welcher der Schwerpunkt der Vorrichtung sich durch entsprechende Anordnung des Gewichts nahe der Körperextremität befindet, führt die Trennung der Gelenkpartner eines Hüftgelenks zumindest dazu, dass der Fuß des Patienten ohne weitere Schmerzbeeinflussung normale Bewegungen durchführen kann.

Mit der erfindungsgemäßen orthopädischen Vorrichtung zur Therapieunterstützung des Hüftgelenks ist am Oberschenkel eine regelmäßige, beispielsweise tägliche Gymnastik durchführbar, bei der das komplette Bein nach Trennung der Gelenkpartner quasi "pendelt". Hierdurch können Personen, die vormals beispielsweise nur noch sehr kleine Schritte gehen konnten wieder einen normalen Bewegungsablauf in der Gehbewegung durchführen, da mittels des verschiebbaren Gewichtselements einerseits eine Zugkraft auf das bewegungseingeschränkte Hüftgelenk und zum anderen eine Druckkraft auf den Oberschenkel nach "hinten" wirkt " so dass sich die Beweglichkeit des Beins praktisch automatisch ergibt. Überdies wird gleichzeitig die Muskulatur des betreffenden Oberschenkels gestärkt, was der Rückkehrt zu einem normalen Bewegungsablauf ebenfalls zuträglich ist. Bei regelmäßiger Übung ist eine Verwendung der Vorrichtung beim Gehen des Patienten nicht notwendig.

Durch die Ausbildung als Manschette lässt sich die die Vorrichtung tragende Stützeinrichtung einfach am Oberschenkel anordnen und anschließend ebenso einfach wieder entfernen.

Bevorzugt umgreift dabei die wenigstens eine Manschette den Oberschenkel zumindest teilweise und liegt dabei zumindest teilweise an diesem an und die Manschettenenden sind mit einem Schnellverschluss, insbesondere einem Klettverschluss versehen, so dass das durch die Vorrichtung an den Oberschenkel zu vermittelnde Moment gut übertragen wird und die Vorrichtung bei Bedarf besonders schnell an- und wieder abgelegt werden kann. Solch ein Schnellverschluss kann beispielsweise auch durch eine an den Manschettenenden befindliche Dorn- oder Faltschließe gebildet sein.

Eine andere vorteilhafte Ausführung der orthopädischen Vorrichtung ist aus Gründen des Tragekomforts zwischen der Stützeinrichtung und dem Oberschenkel mit einer Polsterung versehen.

Die Übertragung des Moments an den Oberschenkel wird bei einer weiteren Ausgestaltung der orthopädischen Vorrichtung dadurch verbessert, dass die Stützeinrichtung durch eine Mehrzahl von Manschetten gebildet ist.

Um der erfindungsgemäßen orthopädischen Vorrichtung eine stabile und gleichzeitig flexible Festlegung an dem Oberschenkel zu ermöglichen kann bzw. können bei einer Ausführung die Manschette(n) der Stützeinrichtung durch ein strapazierfähiges flexibles, insbesondere im wesentlichen einteiliges Band gebildet sein, bei einer weiteren Ausführung können die Manschette bzw. die Manschetten mit wenigstens einem starren und wenigstens einem beweglichen Manschettenteil vorgesehen sein.

Eine gute Anpassbarkeit an die Form des zu beaufschlagenden Oberschenkels wird bei mehrteiligen Manschetten der Stützeinrichtung einer Weiterbildung der orthopädische Vorrichtung dadurch erreicht, dass in einem oder beiden Endbereichen des starren Manschettenteils Öffnungen angeordnet sind, durch welche das zugeordnete bewegliche Manschettenteil zumindest in Gebrauchsstellung der jeweiligen Manschette greift. Ein "festes" Ende des beweglichen Manschettenteils kann so an der einen Öffnung dauerhaft festgelegt sein, das andere bewegliche Ende durch die zugeordnete Öffnung zum Verschluss oder Öffnen der Manschette in einfacher Weise gefädelt und etwa durch übereinander liegende Bereiche des gleichen Endes in Art eines Klettverschlusses geschlossen gehalten sein.

Weiter können bei einer zweckmäßigen Ausführung der orthopädischen Vorrichtung an der Stützenrichtung bei Manschetten mit starren Manschettenteilen ebensolche starren Manschettenteile, die rumpfferner angeordnet sind, insbesondere an derjenigen Manschette, die mit dem Arm des Halteteils versehen sind, eine andere, geringer Größe aufweisen, als rumpfnähere.

Bei einer anderen vorteilhaften Ausführung der orthopädischen Vorrichtung können überdies die starren und beweglichen Manschettenteile mehrerer Manschetten der gleichen Stützeinrichtung jeweils unterschiedlichen Längsflächen der Oberschenkeloberfläche zugeordnet sein, so dass beispielsweise die Manschettenverschlüsse nicht notwendigerweise alle in eine Richtung, also etwa alle nach vorne gerichtet sind, und sich derart eine vorteilhafte Last- und Momentenverteilung am Oberschenkel ergibt.

Zweckmäßigerweise ändern mehrere einer Stützeinrichtung der orthopädische Vorrichtung zugeordnete Manschetten bei Anordnung am Oberschenkel den Abstand zueinander nicht oder zumindest nur unwesentlich, so dass eine definierte Momenteneinleitung erreicht werden kann, und nicht etwa durch unbeabsichtigte Abstandsänderung der Manschetten zueinander verfälschte oder eventuell sogar kontraindizierte Falscheinleitung des Moments stattfänden, weswegen bei einer vorteilhaften Ausführungsform der orthopädischen Vorrichtung die Manschetten der Stützeinrichtung durch das Halteteil und/oder wenigstens ein nicht dem Halteteil zugeordnetes Verbindungselement miteinander verbunden sind.

Die Manschetten der Stützeinrichtung können hierbei also zum einen durch das Halteteil miteinander verbunden sein, welches stangenartig ausgebildet von jeweils wenigstens einer einer Manschette zugeordneten Aufnahme gehalten ist, welche Aufnahmen bevorzugparallel zur Richtung der Längserstreckung des Oberschenkels linear angeordnet sind. Die beabstandet voneinander angeordneten Manschetten können eine effektive Momentenübertragung etwa durch eine gleichmäßige Verteilung über die Länge der Extremität gewährleisten.

Be einer vorteilhaften Weiterbildung der orthopädischen Vorrichtung weist die Stützeinrichtung eine Mehrzahl von strebenartigen Verbindungselementen auf, die mit den Außenflächen der Manschetten verbunden sind und deren eingefasster Querschnitt sich mit zunehmendem Abstand vom Körperrumpf verjüngt und derart der anatomischen Schenkelform in gewissem Maße angepasst ist bzw. folgt. Die Streben können hierfür eine kontinuierlich gebogene bzw. ein oder mehrfach gekrümmte Form aufweisen. Da die Festlegung an der Manschettenaußenfläche vorgesehen ist, sind die Streben derart auf Abstand von dem Oberschenkel gehalten.

Besonders bevorzugt ist hierbei eine Weiterbildung der orthopädischen Vorrichtung, bei der die Stützeinrichtung zwei strebenartige Verbindungselemente aufweist, die in Gebrauchsstellung der Vorrichtung benachbart zu einander gegenüberliegenden Oberflächenbereichen des Oberschenkels angeordnet sind, so dass die Streben weder bei der Anordnung der Vorrichtung bzw. der Manschetten der Stützeinrichtung noch bei therapeutischem Gebrauch der Vorrichtung als hinderlich empfunden werden.

In einer vorteilhaften Ausführung ist das Halteteil der orthopädischen Vorrichtung mehrarmig, insbesondere zweiarmig vorgesehen, wobei ein erster Arm des Halteteils von dem Oberschenkel absteht, wodurch in einfacher Weise eine Übertragung eines Moments durch das Gewichtselement auf den Oberschenkel möglich wird.

Besonders vorteilhaft ist dabei der erste, von dem Oberschenkel abstehende Arm des Halteteils an dem rumpffernen Ende des zweiten Arms angeordnet und weist zumindest mit einer Richtungskomponente in Richtung des Körperrumpfs, so dass die Vorrichtung in gewisser Weise platzsparend angeordnet ist und sowohl den sie tragenden Patienten als auch seine Umgebung bei voller Funktion möglichst wenig beeinträchtigt.

Bei einer anderen Ausführungsform kann es aus Komfortgründen, beispielweise um dem Gewicht der Vorrichtung kurzfristig eine andere Position zuzuweisen, vorgesehen sein, das Halteteil um eine entlang seines zweiten Arms verlaufende Achse verschwenkbar vorzusehen. Bevorzugt wird diese Achse dann durch den zweiten Arm des Halteteils selbst gebildet.

Eine besonders zweckmäßige erfindungsgemäße orthopädische Vorrichtung ist mit einem zweiarmigen Halteteil versehen, dessen Arme in einer bevorzugten Gebrauchsstellung einen Winkel von 55° bis 85°, bevorzugt 70° einschließen und eine zu einem entlang des Oberschenkels verlaufenden Knochen im wesentlichen parallele Ebene aufspannen. Bevorzugt liegt dabei der betroffene Oberschenkelknochen in der durch die Arme des Halteteils aufgespannten Ebene. Bei einer anderen Ausführung kann der erwähnte Winkel von dem einzigen, von dem Oberschenkel abstehenden Arm des Halteteils mit dem Knochen des Oberschenkels eingeschlossen sein.

In einer bevorzugten Ausführung kann das Gewichtselement einen im wesentlichen kreisförmigen Querschnitt aufweisen und insbesondere mit einer zentralen Durchgriffsöffnung versehen sein, durch welche der erste Arm des Halteteils greift, so dass es ohne weiteres an dem Arm verschoben werden und derart das ausgeübte Moment verändert werden kann.

Bevorzugt können demnach bei einer Ausführung der orthopädischen Vorrichtung an dem ersten Arm des Halteteils mehrere Gebrauchsstellungen des Gewichtselements vorgesehen sein, in welcher dieses an dem Arm festlegbar ist. Hierzu kann an dem Halteteil oder dem Gewichtselement ein Festlegemittel angeordnet sein. Ein Verlagerung des Gewichtselements aus einer bereits weiter oben beschriebenen extremitätsnahen Stellung in eine extremitätsferne Stellung führt dazu, dass nicht nur der Fuß normale Bewegungen durchführen kann, sondern vielmehr durch das geänderte Moment der komplette Bewegungsablauf einer Schrittbewegung durch den Patienten schmerzfrei und dabei in aufrechter Haltung ausgeführt werden kann. Ebenso wird in dieser Gebrauchsstellung des Halteteils ein mit Schmerzen verbundenes Kippen des Beckens des Patienten gegen dessen Wirbelsäule erfolgreich verhindert.

Um eine Vielzahl möglicher Stellungen der orthopädischen Vorrichtung an dem Oberschenkel zu ermöglichen kann zwischen wenigstens zwei Armen des Halteteils außerdem bei einer Weiterbildung ein Gelenk angeordnet sein, welches die Verschwenkbarkeit eines ersten Arms gegenüber einem weiteren in wenigstens einer Ebene gestattet.

Die Erfindung wird nachstehend anhand Ausführungsbeispielen in der Zeichnung näher beschrieben. Es zeigen hierbei in schematisierter Darstellung die
- Fig.1: eine Seitenansicht eines ersten Ausführungs- beispiels der erfindungsgemäßen orthopädischen
- Fig.2: Vorrichtung mit Stützeinrichtung und Halteteil entlang einer durch einen Arm des Halteteils gebildeten Achse; eine Seitenansicht der orthopädischen Vorrich- tung aus der Fig.1 mit gegenüber der Dar- stellung der Fig.1 verschobenem Gewicht;
- Fig.3: eine perspektivische Seitensicht der ortho- pädischen Vorrichtung der Fig.1 und 2 mit ver- schiedenen Gebrauchsstellungen des Gewichts an dem Halteteil;
- Fig.4: eine Seitenansicht der orthopädischen Vor- richtung zur Verdeutlichung des zwischen den Armen des Halteteils gebildeten Winkels;
- Fig.5,6: eine Seitenansicht der Anordnung der Ausfüh- rungsform der Fig. 1 bis 4 der orthopädischen Vorrichtung an einem rechten bzw. linken Ober- schenkel eines Patienten;
- Fig.7: eine Seitenansicht eines zweiten Ausführungs- beispiels der erfindungsgemäßen orthopädischen Vorrichtung mit Stützeinrichtung und Halteteil entlang einer durch einen gedachten Oberschen- kel gebildeten Achse;
- Fig.8: eine Seitenansicht der orthopädischen Vorrich- tung aus der Fig.7 mit gegenüber der Dar- stellung der Fig.7 verschobenem Gewicht und ausgefädelten Verschlussenden;
- Fig.9: eine perspektivische Seitensicht der ortho-
- Fig.10: pädischen Vorrichtung der Fig.7 und 8 mit ver- schiedenen Gebrauchsstellungen des Gewichts an dem Halteteil; eine Seitenansicht der Anordnung der Ausfüh- rungsform der orthopädischen Vorrichtung der Fig.7 bis 9 an einem linken Oberschenkel eines Patienten;

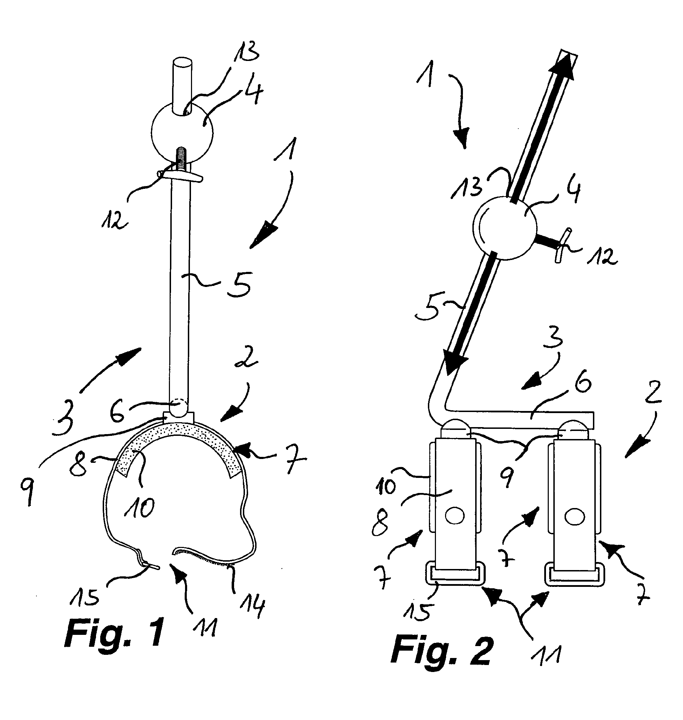

Die Fig.1 zeigt zunächst eine im Ganzen mit 1 bezeichnete orthopädische Vorrichtung zur Therapieunterstützung bei Gelenkbeschwerden des Hüftgelenks. Diese Vorrichtung 1 kann an einem in der Fig.1 nicht dargestellten Oberschenkel angeordnet werden, an dessen einem Ende sich einer der Gelenkpartner des betroffenen Gelenks befindet und ist mit einer Stützeinrichtung 2 und einem mit dieser verbundenen Halteteil 3 versehen. Das Halteteil 3 ist mit einem Gewicht in Form eines Gewichtselements 4 versehen, welches in wenigstens einer Gebrauchsstellung ein Moment auf den Oberschenkel ausübt, das die Gelenkpartner trennt. In der Fig.1 ist dabei die orthopädische Vorrichtung 1 mit Stützeinrichtung 2 und Halteteil 3 in einer Ansicht entlang einer durch einen Arm 6 des Halteteils 3 gebildeten Achse dargestellt, so dass der andere Arm 5 des Halteteils 3 mit dem daran angeordneten Gewichtselement 4 aus der Blattebene auf den Betrachter hin weist.

Zu erkennen ist in der Fig.1 weiter die dem Betrachter zugewandte Manschette 7 der Stützeinrichtung 2, welche durch ein flexibles Band 8 gebildet ist, in dessen ungefährer Mitte sich eine Aufnahme 9 zur Anordnung eines Bereichs des Arms 6 der Halteteils 2 befindet. Durch die Manschette 7 ist die orthopädische Vorrichtung 1 mit dem Oberschenkel lösbar verbindbar. Bei Verbindung mit dem Oberschenkel umgreift die Manschette 7 die Körperextremität und die Enden des Bandes 8 sind zur Festlegung der Manschette 7 mit einem Schnellverschluss 11 versehen. Der diesen Schnellverschluss 11 bildende Klettverschluss ist dabei so ausgebildet, dass ein Ende des Bandes mit einem Klettbereich 14 versehen ist und dieses Ende durch eine an dem anderen Ende des Bandes 8 angebrachte Schlaufe oder Lasche 15 greift, um nachfolgend an der eigenen Bandseite festgelegt zu werden. An der dem nicht dargestellten Oberschenkel zugewandten Seite des Bandes 8 der Manschette 7 befindet sich außerdem ein Polster 10 zur Verbesserung des Komforts beim Tragen der orthopädischen Vorrichtung.

Besser als in der Fig.1 ist in der Ansicht der Fig.2 erkennbar, dass die Stützeinrichtung 2 zwei Manschetten 7 zur Verbindung der orthopädischen Vorrichtung 1 mit dem Oberschenkel aufweist und die Manschetten 7 durch einen Arm 6 des Halteteils 3 miteinander verbunden sind, wobei der Arm 6 an Aufnahmen 9 an den Manschetten 7 angeordnet ist.

Darüber hinaus lässt sich der Fig.2 entnehmen, dass das Halteteil 3 mehrarmig, im vorliegenden Fall zweiarmig vorgesehen ist und ein erster Arm 5 des Halteteils 3 von dem nicht dargestellten Oberschenkel absteht. Dieser erste Arm 5 ist dabei an dem körperfernen Ende des zweiten Arms 6 angeordnet und weist in Richtung des Körperrumpfs. Außerdem wird in Verbindung mit der Fig.2 und den dort gezeigten Pfeilen deutlich, dass das Gewichtselement 4 an dem Arm 5 verlagerbar ist und überdies mit einem Festlegemittel 12 versehen ist, das in angezogenem Zustand ein Verschieben des Gewichtselements 4 verhindert. Wie schon in der Fig.1 ist das Gewichtselement 4 kugelförmig ausgebildet und weist eine Durchgriffsöffnung 13 auf, durch welche der Arm 5, an welchem das Gewichtselement 4 verlagerbar ist, greift.

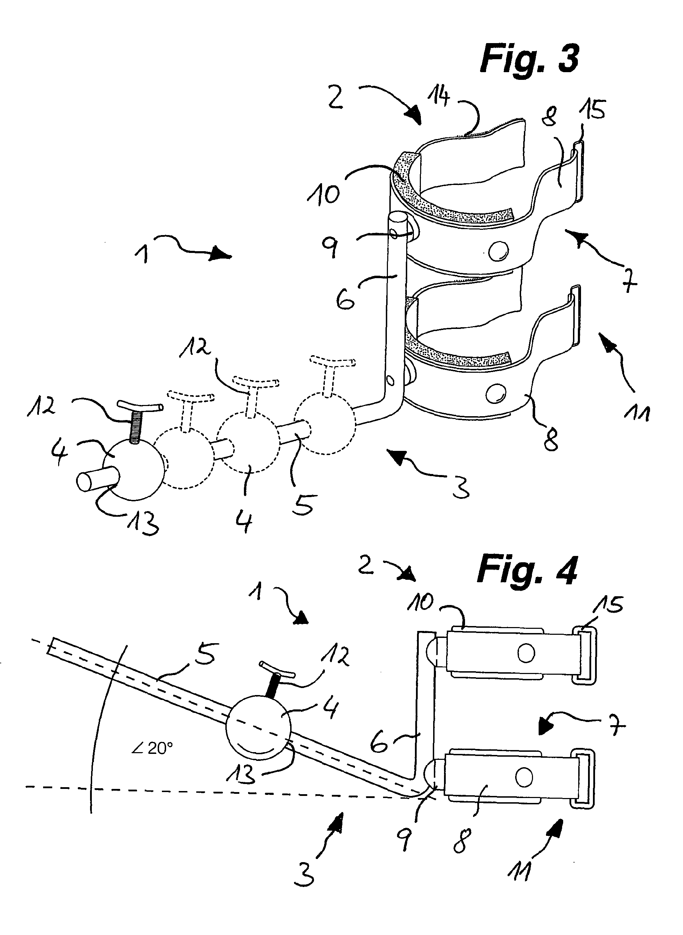

In der Fig.3 ist die orthopädische Vorrichtung 1 in einer perspektivischen Ansicht mit einer Mehrzahl möglicher Gebrauchstellungen des Gewichtselements 4 des Halteteils 3 zu erkennen, wobei diese mehreren Gebrauchsstellungen des gleichen Gewichtselements 4 durch Strichelungen angedeutet sind.

Die Seitenansicht der Fig.4 verdeutlicht, dass bei dem vorliegenden Ausführungsbeispiel der erste Arm 5 und der zweite Arm 6 des Halteteils 3 der orthopädischen Vorrichtung 1 einen Winkel von 70° einschließen, weswegen, wie dargestellt, der von dem ersten Arm 5 mit dem Lot auf den zweiten Arm eingeschlossene Winkel 20° beträgt.

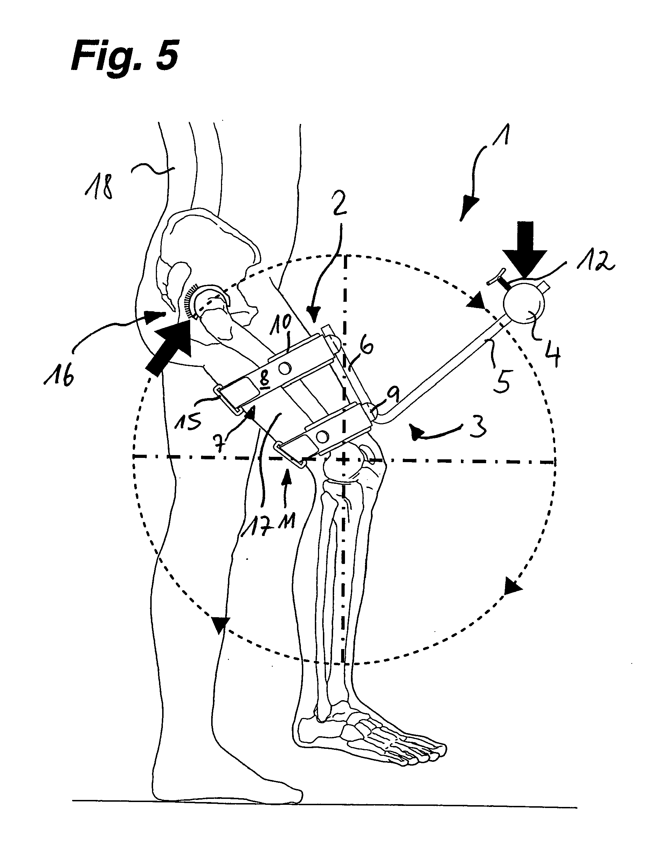

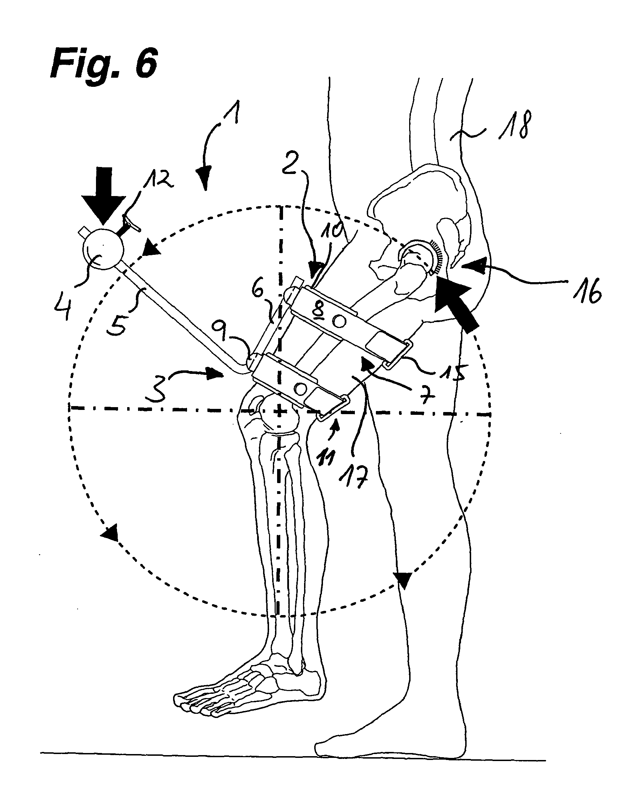

Die Fig.5 und 6 zeigen die orthopädische Vorrichtung 1 zur Therapieunterstützung von Gelenkbeschwerden des Hüftgelenks, die zum einen an einem rechten (Fig.5), zum anderen an einem linken Oberschenkel 17 (Fig.6) eines Patienten angeordnet ist und daher zur Einwirkung auf Beschwerden des jeweils zugeordneten Hüftgelenks 16 vorgesehen ist. An dem einen Ende des jeweiligen Oberschenkels 17 befindet sich daher einer der Gelenkpartner des betroffenen Gelenks 16, hier der Kopf des Oberschenkelknochens. Außerdem weist die Vorrichtung 1 eine Stützeinrichtung 2 und ein zweiarmiges Halteteil 3 auf. Das Halteteil ist mit einem Gewicht in Form eines Gewichtselements 4 versehen, das in einer Gebrauchsstellung ein Moment auf den Oberschenkel 17 ausübt, welches die Partner des betreffenden Gelenks 16, hier also die Gelenkpfanne des Hüftknochens und den Kopf des Oberschenkelknochens, trennt.

Die Stützeinrichtung 2 der Vorrichtung 1 ist durch eine Mehrzahl von Manschetten 7 gebildet, die durch einen Arm 6 des Halteteils 3 miteinander verbunden sind, wobei die Manschetten 7 den Oberschenkel 17 an dieser anliegend umgreifen und die Enden der Manschetten 7 mit einem Schnellverschluss 11 versehen sind, der die Enden verbindet und aneinander hält. Zwischen dem flexiblen Band 8 der Manschette 7 und dem Oberschenkel 17 ist außerdem jeweils ein Polster 10 angeordnet.

Das Halteteil 3 der orthopädischen Vorrichtung 1 ist in den Fig.5 und 6 zweiarmig vorgesehen, wobei der erste Arm 5 des Halteteils von dem Oberschenkel 17 absteht, an dem rumpffernen Ende des zweiten Arms 6 angeordnet ist und in Richtung des Körperrumpfs 18 weist. Die Arme 5, 6 des zweiarmigen Halteteils 3 der Vorrichtung 1 schließen in der dargestellten Gebrauchsstellung einen Winkel von 70° ein und spannen eine zu dem entlang des Oberschenkels 17 verlaufenden Oberschenkelknochen eine im wesentlichen parallele Ebene auf, vorliegend liegt der Oberschenkelknochen sogar in dieser Ebene.

Das das Gewicht bildende, an dem Arm 5 verlagerbare, kugelförmige Gewichtselement 4 ist in den Fig.5 und 6 mittels des Festlegemittels 12 in einer extremitätsfernen Stellung festgelegt, so dass ein größeres Moment auf den Oberschenkel 17 ausgeübt wird, als in einer extremitätsnahen Gebrauchsstellung des Gewichtselements 4.

Durch das mittels des Gewichtselements 4 ausgeübte Moment werden die Gelenkpartner des Gelenks 16 getrennt, wobei die extremitätsferne Stellung des Gewichtselements 4 dazu führt, dass das gezeigte Kniegelenk und die untere Extremität samt Fuß keinerlei Bewegungseinschränkungen durch an dem Gelenk 16 verursachten Schmerzen unterliegt.

Außerdem kann durch das geänderte Moment der komplette Bewegungsablauf einer Schrittbewegung durch den Patienten schmerzfrei und dabei in aufrechter Haltung ausgeführt werden. Ebenso wird in dieser Gebrauchsstellung des Halteteils 3 ein mit Schmerzen verbundenes Kippen des Beckens des Patienten gegen dessen Wirbelsäule erfolgreich verhindert.

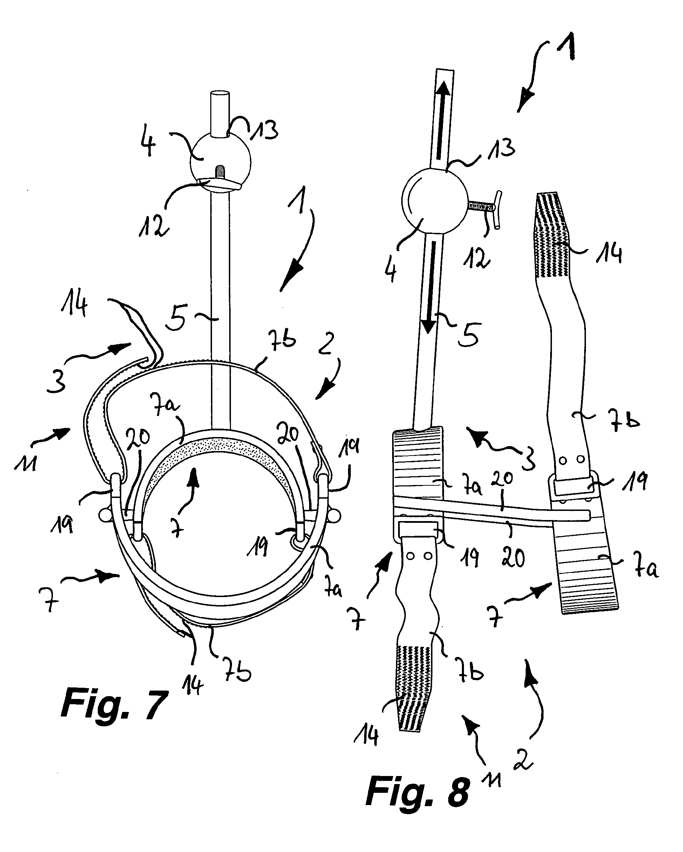

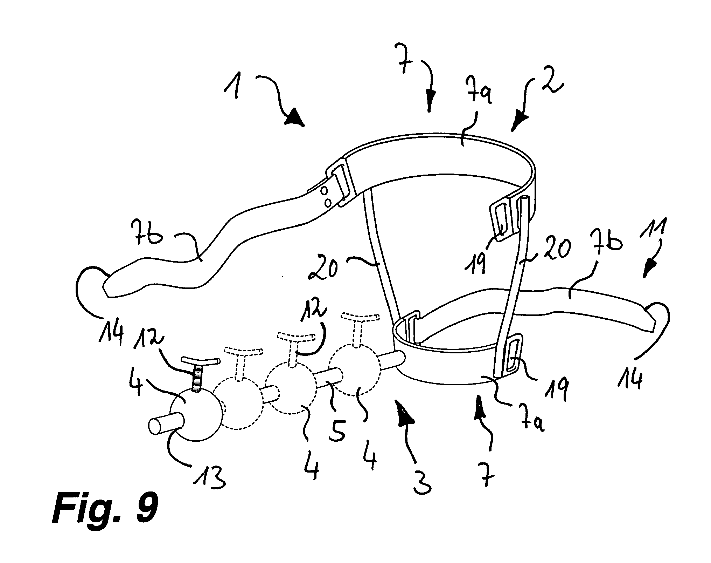

In den Fig.7 bis 10 ist eine zweite Ausführungsform der erfindungsgemäßen orthopädischen Vorrichtung 1 zu erkennen, die der ersten aus den Fig.1 bis 6 strukturell ähnlich ist, aber auch einige Unterschiede aufweist. So ist die in den Fig.7 bis 10 erkennbare Vorrichtung mit einer Stützeinrichtung 2 mit zwei Manschetten 7 versehen, die jeweils ein starres und bewegliches Manschettenteil 7a, 7b aufweisen. Erkennbar ist in den erwähnten Figuren weiter, dass jeweils in den Endbereichen des starren Manschettenteil 7a Ösen als Öffnungen 19 angeordnet sind. Mit einem seiner Endbereich ist das jeweilige bewegliche Manschettenteil 7b der Manschetten 7 an einer der ösenartigen Öffnungen 19 um eine quer zur Längserstreckung des beweglichen Manschettenteils 7b weisenden Schwenkachse beweglich festgelegt. Das andere, freie Ende des beweglichen Manschettenteils 7a lässt sich durch die freie Öffnung 19 fädeln und fixiert mittels des in seinem Endbereich befindlichen Klettbereichs 14 die Manschette 7 an dem in den Fig. 7 bis 9 nicht dargestellten Oberschenkel 17. Es ist in den Fig. 7 bis 10 auch zu erkennen, dass das starre Manschettenteil 7a der rumpfferner anzuordnenden Manschette 7 der Stützeinrichtung 2 eine geringere Größe aufweist, als das rumpfnähere starre Manschettenteil 7a.

Den Fig.7 bis 10 gut zu entnehmen ist der Umstand, dass das Halteteil 3 mit dem verlagerbaren Gewichtselement 4 nur einen Arm 5 aufweist, der an dem rumpfferneren der beiden Manschetten 7 der Stützeinrichtung 2 angeordnet ist und von dieser von dem Oberschenkel 17 absteht. Der Arm 5 steht dabei nicht senkrecht von dem Oberschenkel 17 ab, vielmehr schließt er mit dem in Richtung des Rumpfs weisenden Bereich des Oberschenkels einen Winkel ein, der kleiner als 90° ist. Weiter lässt sich den Fig. 7 bis 10 entnehmen, dass die starren und beweglichen Manschettenteile 7a, 7b der beiden der Stützeinrichtung 2 zugeordneten Manschetten 7 jeweils unterschiedlichen Längsflächen der Oberfläche des Oberschenkels 17 zugeordnet sind und daher bei der rumpfferneren Manschette 7 das starre Manschettenteil 7a an der Vorderseite des Oberschenkels liegt, das bewegliche Manschettenteil 7b an der Rückseite, bei der rumpfnäheren Manschette 7 verhält es sich umgekehrt.

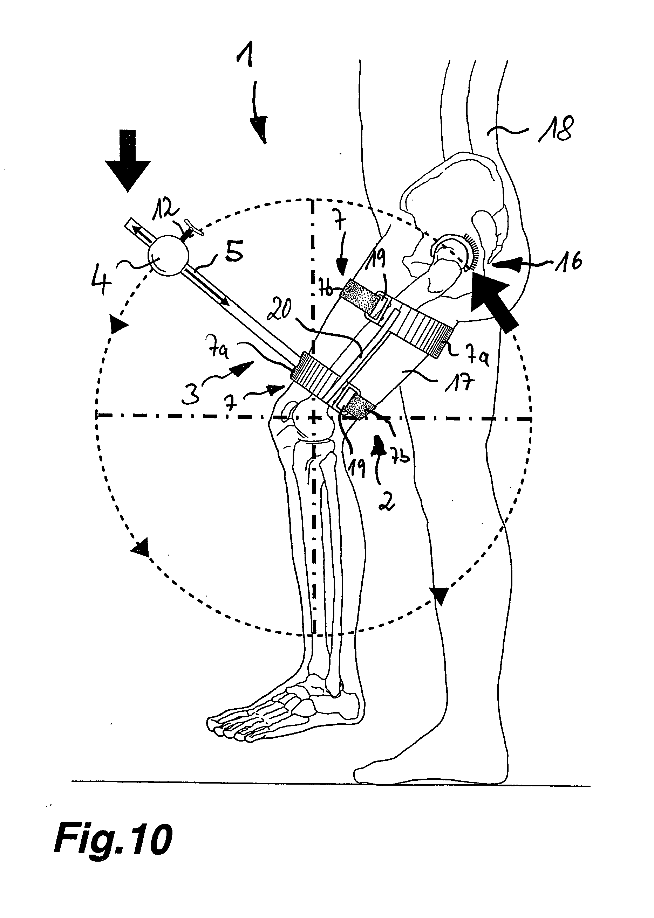

Im Unterschied zu den Fig.1 bis 6 sind bei der Ausführungsform der Fig.7 bis 10 die Manschetten 7 der Stützeinrichtung 2 nicht durch das Halteteil 3 bzw. einen Arm 6 desselben, sondern durch zwei nicht dem Halteteil 3 zugeordnete, strebenartige Verbindungselemente 20 miteinander verbunden. Diese sind in ihrem Endbereichen jeweils mit den Außenflächen der Manschetten 7 verbunden, wobei sich ihr eingefasster Querschnitt mit zunehmendem Abstand vom Körperrumpf 18 verjüngt. Die beiden strebenartigen Verbindungselemente 20 sind hierbei benachbart zu einander gegenüberliegenden Oberflächenbereichen des Oberschenkels 17, etwa in der Fig.10 auf der linken und rechten Seite des Oberschenkels angeordnet. Hierbei weisen sie eine kontinuierliche Krümmung auf, um den sich ändernden Querschnitt des Oberschenkels 17 nachzuvollziehen, zu dem sie, ebenfalls in der Fig.10 zu erkennen, im wesentlichen parallel verlaufen.

Demnach betrifft die vorstehend beschriebene Erfindung also eine orthopädische Vorrichtung 1 zur Therapieunterstützung bei Gelenkbeschwerden des Hüftgelenks, welche Vorrichtung 1 an demjenigen dem Hüftgelenk zugeordneten Oberschenkel 17 anordenbar ist, an dessen einem Ende sich einer der Gelenkpartner des betroffenen Hüftgelenks befindet, mit einer Stützeinrichtung 2, die durch wenigstens eine mit dem Oberschenkel 17 lösbar anordenbare Manschette 7 gebildet ist.

Um eine einfach handhabbare orthopädische Vorrichtung 1 zur Verfügung zu haben, die bei einer durch Gelenkbeschwerden beeinträchtigten Person eine weitgehend schmerzfreie Verbesserung von Bewegungsabläufen, insbesondere von gelenkfernen Gliedmaßen, ermöglicht, wird vorgeschlagen, bei einer solchen Vorrichtung 1 die Stützeinrichtung 2 mit einem Halteteil 3 zu verbinden, welches ein Gewichtselement 4 aufweist, dessen in einer Gebrauchsstellung auf den Oberschenkel 17 ausgeübtes Moment die Gelenkpartner des Hüftgelenks trennt und das Halteteil 3 mit einem ersten, von dem Oberschenkel 17 abstehenden Arm 5 zu versehen, entlang dessen das Gewichtselement 4 verlagerbar angeordnet ist.

## Patentansprüche

1. Orthopädische Vorrichtung zur Therapieunterstützung bei Gelenkbeschwerden des Hüftgelenks (16), welche Vorrichtung an demjenigen dem Hüftgelenk (16) zugeordneten Oberschenkel (17) anordenbar ist, an dessen einem Ende sich einer der Gelenkpartner des betroffenen Hüftgelenks (16) befindet, mit einer Stützeinrichtung (2), die durch wenigstens eine mit dem Oberschenkel lösbar (17) anordenbare Manschette (7) gebildet ist, **dadurch gekennzeichnet, dass** die Stützeinrichtung (2) mit einem Halteteil (3) verbunden ist, welches ein Gewichtselement (4) aufweist, dessen in einer Gebrauchsstellung auf den Oberschenkel (17) ausgeübtes Moment die Gelenkpartner des Hüftgelenks (16) trennt, und dass das Halteteil (3) mit einem ersten, von dem Oberschenkel (17) abstehenden Arm (5) versehen ist, entlang dessen das Gewichtselement (4) verlagerbar angeordnet ist.

2. Orthopädische Vorrichtung nach Anspruch 1, **dadurch gekennzeichnet, dass** die wenigstens eine Manschette (7) den Oberschenkel (17) zumindest teilweise an diesem anliegend umgreift und die Enden der Manschetten (7) mit einem Schnellverschluss (11) versehen sind.

3. Orthopädische Vorrichtung nach Anspruch 2, **dadurch gekennzeichnet, dass** der Schnellverschluss (11) durch einen Klettverschluss gebildet ist.

4. Orthopädische Vorrichtung nach Anspruch 2 oder 3, **dadurch gekennzeichnet, dass** zwischen der Stützeinrichtung (2) und dem Oberschenkel (17) eine Polsterung (10) vorgesehen ist.

5. Orthopädische Vorrichtung nach einem der Ansprüche 1 bis 4, **dadurch gekennzeichnet, dass** die Stützeinrichtung (2) durch eine Mehrzahl von Manschetten (7) gebildet ist.

6. Orthopädische Vorrichtung nach einem der vorhergehenden Ansprüche, **dadurch gekennzeichnet, dass** die Manschette bzw. die Manschetten (7) der Stützeinrichtung (2) als strapazierfähiges flexibles Band (8) oder mit wenigstens einem starren und wenigstens einem beweglichen Manschettenteil (7a, 7b) vorgesehen sind.

7. Orthopädische Vorrichtung nach Anspruch 6, **dadurch gekennzeichnet, dass** in den beiden Endbereichen des starren Manschettenteils (7a) Öffnungen (19) angeordnet sind, durch welche das zugeordnete bewegliche Manschettenteil (7b) zumindest in Gebrauchsstellung der jeweiligen Manschette (7) greift.

8. Orthopädische Vorrichtung nach Anspruch 6 oder 7, **dadurch gekennzeichnet, dass** rumpfferner angeordnete, starre Manschettenteile (7a) einer insbesondere mit dem ersten Arm (5) des Halteteils (3) versehenen Manschette (7), eine andere, geringere Größe aufweisen, als rumpfnähere.

9. Orthopädische Vorrichtung nach einem der Ansprüche 6 bis 8, **dadurch gekennzeichnet**, die starren und beweglichen Manschettenteile (7a, 7b) mehrerer Manschetten (7) der gleichen Stützeinrichtung (2) jeweils unterschiedlichen Längsflächen der Oberfläche des Oberschenkels (17) zugeordnet sind.

10. Orthopädische Vorrichtung nach einem der vorhergehenden Ansprüche, **dadurch gekennzeichnet, dass** die Manschetten (7) der Stützeinrichtung (2) durch das Halteteil (3) und/oder wenigstens ein nicht dem Halteteil (3) zugeordnetes Verbindungselement (20) miteinander verbunden sind.

11. Orthopädische Vorrichtung nach Anspruch 10, **dadurch gekennzeichnet, dass** die Stützeinrichtung (2) eine Mehrzahl von strebenartigen Verbindungselementen (20) aufweist, die mit den Außenflächen der Manschetten (7) verbunden sind und deren eingefasster Querschnitt sich mit zunehmendem Abstand vom Körperrumpf (18) verjüngt.

12. Orthopädische Vorrichtung nach Anspruch 10 oder 11, **dadurch gekennzeichnet, dass** die Stützeinrichtung (2) zwei strebenartige Verbindungselemente (20) aufweist, die in Gebrauchsstellung der Vorrichtung (1) benachbart zu einander gegenüberliegenden Oberflächenbereichen des Oberschenkels (17) angeordnet sind.

13. Orthopädische Vorrichtung nach einem der vorhergehenden Ansprüche, **dadurch gekennzeichnet, dass** das Halteteil (3) mehrarmig vorgesehen ist.

14. Orthopädische Vorrichtung nach Anspruch 13, **dadurch gekennzeichnet, dass** der erste, von dem Oberschenkel (17) abstehende Arm (5) des Halteteils (3) an dem rumpffernen Ende eines zweiten Arms (6) angeordnet ist und in Richtung des Körperrumpfs (18) weist.

15. Orthopädische Vorrichtung nach Anspruch 13 oder 14, **dadurch gekennzeichnet, dass** das Halteteil (3) um eine entlang seines zweiten Arms (6) verlaufende Achse verschwenkbar vorgesehen ist.

16. Orthopädische Vorrichtung nach einem der vorhergehenden Ansprüche, **dadurch gekennzeichnet, dass** die Arme (5, 6) eines zweiarmigen Halteteils (3) oder der erste Arm (5) eines einarmigen Halteteils (3) mit dem Knochen des Oberschenkels (17) in einer Gebrauchsstellung einen Winkel zwischen 55° und 85°, insbesondere einen Winkel von 70° einschließen und eine zu einem entlang des Oberschenkels (17) verlaufenden Knochen parallele Ebene aufspannen.

17. Orthopädische Vorrichtung nach einem der vorhergehenden Ansprüche, **dadurch gekennzeichnet, dass** das Gewichtselement (4) einen kreisförmigen Querschnitt aufweist und mit einer zentralen Durchgriffsöffnung (13) versehen ist, durch welche der erste Arm (5) des Halteteils (3) greift.

18. Orthopädische Vorrichtung nach einem der vorhergehenden Ansprüche, **dadurch gekennzeichnet, dass** an dem ersten Arm (5) des Halteteils (3) mehrere Gebrauchsstellungen des Gewichtselements (4) vorgesehen sind, in welcher dieses an dem Arm (5) festlegbar ist.

19. Orthopädische Vorrichtung nach einem der vorhergehenden Ansprüche, **dadurch gekennzeichnet, dass** zwischen wenigstens zwei Armen (5, 6) des Halteteils (3) ein Gelenk angeordnet ist, welches die Verschwenkbarkeit eines ersten Arms (5) gegenüber einem weiteren (6) in wenigstens einer Ebene gestattet.
